# FASCICULE DE BREVET EUROPEEN

(11) **EP 0 993 438 B1**
(45) Date de publication et mention de la délivrance du brevet: **12.06.2002**
(21) Numéro de dépôt: 98935082.2
(22) Date de dépôt: 02.07.1998
(51) Int. Cl.: C07C 249/06, C07C 251/44

(54) **PHOTONITROSATION DE CYCLODODECANE DANS LE CHLOROFORME EN MILIEU QUASI-ANHYDRE**
PHOTONITROSIERUNG VON CYCLODODECAN IN CHLOROFORM IN EINEM MILIEU MIT EINGESCHRANKTEM WASSERGEHALT
PHOTONITROSATION OF CYCLODODECANE IN CHLOROFORM IN QUASI-ANHYDROUS MEDIUM

(30) Priorité: 02.07.1997 FR 9708357
(43) Date de publication de la demande: 19.04.2000
(73) Titulaire: Atofina, 92800 Puteaux (FR)
(72) Inventeur: OLLIVIER, Jean, F-64260 Arudy (FR); DRUTEL, Damien, F-64000 Pau (FR)
(86) Numéro de dépôt international: FR9801415
(87) Numéro de publication internationale: WO9901424

(56) Documents cités:
- EP-A- 0 798 290
- DE-B- 1 240 074
- GB-A- 1 095 916
- GB-A- 1 136 747

## Description

La présente invention a trait au domaine des polymères du type polyamide 12 obtenus par polymérisation du lauryllactame. Elle concerne plus particulièrement un procédé de préparation de l'oxime de la cyclododécanone (intermédiaire réactionnel du lauryllactame) qui consiste à effectuer la photonitrosation du cyclododécane dans le chloroforme en milieu quasi-anhydre.

La production d'oximes cycloaliphatiques par la voie photochimique est connue depuis de nombreuses années. De manière classique, on fait réagir photochimiquement un cycloalcane avec un agent nitrosant, en particulier le chlorure de nitrosyle, en présence d'un excès de chlorure d'hydrogène, à des températures variant de -30°C à +40°C. L'oxime ainsi formée, sous forme de chlorhydrate, est extraite du milieu réactionnel par exemple au moyen d'un acide minéral fort. Cette oxime est ensuite soumise à une étape de transposition par réaction de Beckmann pour former le lactame correspondant.

La production de lauryllactame dans un procédé industriel fonctionnant en continu est sous la dépendance de plusieurs facteurs limitants.

Au niveau de l'étape de photonitrosation, les paramètres importants directement liés à la réaction photochimique sont :
- la nature du milieu dans lequel le cyclododécane est nécessairement solubilisé puisqu'à la température normale de réaction ce dernier est solide (voir par exemple DE-A-1 240 074 et GB-A-1 095 916),
- la nature du solvant à introduire dans le milieu de réaction pour augmenter la solubilité de l'oxime et prévenir ainsi son dépôt sur la paroi des lampes d'irradiation (voir par exemple FR-A-1 335 823, FR-A-1 553 268, GB-A-1 136 747 et DE 1 136 747), et
- l'influence des solvants précités sur l'homogénéité et la transparence du milieu réactionnel qui agissent directement sur l'efficacité des photons lumineux.

Dans l'état de la technique précité, on ne trouve aucune référence à l'influence de la teneur en eau dans le milieu réactionnel.

La demanderesse a maintenant trouvé que la limitation de la teneur en eau dans l'étape industrielle de photonitrosation permet d'augmenter notablement le rendement en oxime de la cyclododécanone, et, par voie de conséquence, d'augmenter la rentabilité des étapes qui se situent en aval, notamment la transposition de Beckmann et le recyclage de l'acide utilisé dans cette dernière étape.

La présente invention a donc pour objet un procédé de préparation d'oxime de la cyclododécanone qui consiste à faire réagir photochimiquement le cyclododécane solubilisé dans le chloroforme, un agent nitrosant et du chlorure d'hydrogène dans un milieu quasi-anhydre, c'est-à-dire dans des conditions telles que la teneur en eau dans le milieu réactionnel n'excède pas 1000 ppm.

Le procédé selon l'invention est mis en oeuvre de manière conventionnelle et dans des conditions de réaction connues pour la nitrosation photochimique de composés cycloaliphatiques.

Plus précisément, pour mettre en oeuvre le procédé selon l'invention, on dilue le cyclododécane dans le chloroforme, on sature la solution avec du chlorure d'hydrogène gazeux, on y ajoute l'agent nitrosant et finalement on irradie avec de la lumière.

Le taux d'humidité de chaque réactif est choisi de manière à ce que la teneur globale en eau dans le milieu réactionnel soit comprise entre 50 et 1000 ppm, et de préférence 250 et 600 ppm.

La réaction est généralement réalisée dans un réacteur et l'introduction des différents réactifs est effectuée en continu.

La concentration de cyclododécane dans le chloroforme est généralement comprise entre 0,1 et 35 % en poids et de préférence 20 et 30 % en poids.

L'agent nitrosant est généralement choisi parmi le chlorure de nitrosyle, un mélange d'oxyde nitrique et de chlore, ou des composés susceptibles de former du chlorure de nitrosyle dans le milieu réactionnel, par exemple des alkyl nitrites réagissant avec le chlorure d'hydrogène. De préférence, on utilise le chlorure de nitrosyle.

On règle l'addition de l'agent nitrosant de manière que sa concentration dans le milieu réactionnel soit comprise entre 0,1 et 25 g/l et de préférence 1 et 2 g/l.

Le chlorure d'hydrogène est généralement introduit sous la forme d'un gaz anhydre, en excès par rapport à l'agent nitrosant. De préférence, il est mis en oeuvre à saturation de la solution de cyclododécane.

L'irradiation est réalisée au moyen d'une ou plusieurs lampes à vapeur de mercure ou de sodium émettant des radiations de longueur d'onde comprise entre 500 et 700 nm et de préférence 565 et 620 nm.

La réaction est généralement effectuée à une température comprise entre - 20 et +40°C et de préférence +10 et +20°C.

On opère en général sous une agitation vigoureuse, par exemple au moyen d'une ou plusieurs pompes de recirculation.

La concentration de l'oxime de la cyclododécanone dans le milieu réactionnel ne doit généralement pas dépasser 15 % en poids pendant la photonitrosation.

Le mélange réactionnel irradié est soutiré en continu et il est soumis à une extraction avec un acide fort, de préférence de l'acide sulfurique ayant une concentration supérieure à 80 % en poids, à une température comprise entré 10 et 50°C, de préférence de l'ordre de 20 à 25°C.

L'oxime de la cyclododécanone ainsi formée est ensuite soumise à une étape de transposition de Beckmann en présence d'acide sulfurique pour former le lauryllactame.

Les exemples qui suivent permettent d'illustrer l'invention.

### EXEMPLE 1

### a - Photonitrosation

Dans un réacteur de deux litres (volume utile) équipé d'une lampe à vapeur de sodium ayant une puissance de 400 watts et émettant un maximum de radiations au voisinage de 595 nm, on introduit en continu une solution de cyclododécane dans le chloroforme (450 g/l ; 1 1/h), de l'acide chlorhydrique gazeux anhydre à saturation et du chlorure de nitrosyle. Le débit de chlorure de nitrosyle est réglé de manière à ce que la concentration dans le réacteur soit maintenue à 2 g/l de milieu réactionnel. La teneur en eau dans le milieu de réaction est égale à 300 ppm.

Les effluents gazeux issus du réacteur sont dirigés vers un condenseur (récupération du solvant) et un barboteur contenant une solution de soude (piégeage de l'acide chlorhydrique).

Le milieu est soutiré en continu à raison de 1,1 l/h environ.

En régime stationnaire, on forme 0,63 mole/h d'oxime de la cyclododécanone, 0,023 mole/h de monochlorocyclododécane, 0,0105 mole/h d'oxime de chlorocyclododécanone et 0,001 mole/h de dichlorocyclododécane.

Le nombre de moles de cyclododécane converti en une heure est égal à 0,670.

La sélectivité molaire en oxime de la cyclododécanone est égale à 0,94 calculée sur la base du cyclododécane ayant réagi.

Dans ces conditions, la productivité horaire en oxime est égale à 1295 g/kW (parcours optique de la lampe : 6,7 cm ; rendement énergétique = 24 %).

### b - Transposition de Beckmann

Le milieu réactionnel soutiré à l'étape a- est traité par de l'acide sulfurique à 98,5 % pour en extraire l'oxime de la cyclododécanone. Après décantation, la solution sulfurique récupérée contient 36 % en poids de l'oxime.

Dans 100 g d'acide sulfurique à 98,5 % maintenu à 155°C et sous agitation, on introduit en une heure 200 g de la solution sulfurique d'oxime précitée. Le mélange est ensuite porté à 160°C pendant 30 minutes.

On récupère 71,28 g de lauryllactame (rendement molaire : 99 %).

Le rendement molaire global (photonitrosation + transposition) est égal à 93 %.

### EXEMPLE 2 (COMPARATIF)

### a - Photonitrosation

On procède dans les conditions de l'exemple 1 modifié en ce que la solution de cyclododécane dans le chloroforme contient 2000 ppm d'eau. Une partie de cette eau est maintenue en suspension dans la solution par agitation dans le dispositif de mélange du cyclododécane et du chloroforme situé en amont de l'alimentation du réacteur.

En phase stationnaire, on forme 0,62 mole/h d'oxime de la cyclododécanone, 0,0226 mole/h de monochlorocyclododécane, 0,0103 mole/h d'oxime de chlorocyclododécanone et 0,00098 mole de dichlorocyclododécane.

Le nombre de moles de cyclododécane converti en une heure est égal à 0,667.

Le rendement molaire en oxime est de 0,929.

### b - Transposition de Beckmann

On procède dans les conditions de l'exemple 1. La solution sulfurique obtenue après l'extraction contient 35 % en poids de l'oxime.

Lorsque la réaction est terminée, on récupère 67,9 g de lauryllactame (rendement molaire : 97 %).

Le rendement molaire global est égal à 90,1 %.

### EXEMPLE 3 (COMPARATIF)

### a - Photonitrosation

On procède dans les conditions de l'exemple 1 modifié en ce que l'on introduit en outre dans le réacteur, en continu, une solution aqueuse d'acide sulfurique à 90 % de sorte que le volume injecté représente 10 % du volume total de milieu réactionnel.

Le milieu réactionnel soutiré en continu est décanté. On récupère 0,43 mole/h d'oxime de la cyclododécanone et 0,011 mole/h d'oxime de chlorocyclododécanone dans la phase aqueuse, 0,016 mole/h de monochlorocyclododécane et 0,0005 mole/h de dichlorocyclododécane dans la phase organique.

Le nombre de moles de cyclododécane converti en une heure est égal à 0,495.

La sélectivité molaire en oxime de la cyclododécanone est égale à 0,875 calculée sur la base du cyclododécane ayant réagi.

Dans ces conditions, la productivité horaire en oxime est égale à 890 g/kW.

### b - Transposition de Beckmann

On procède dans les conditions de l'exemple 1. La solution sulfurique obtenue après l'extraction contient 30 % en poids de l'oxime.

A l'issue de la transposition, on récupère 68,4 g de lauryllactame (rendement molaire : 97 %).

Le rendement molaire global est égal à 84,8 %.

## Revendications

1. Procédé de préparation d'oxime de la cyclododécanone qui consiste à faire réagir photochimiquement le cyclododécane solubilisé dans le chloroforme, un agent nitrosant et du chlorure d'hydrogène dans des conditions telles que la teneur en eau dans le milieu réactionnel n'excède pas 1000 ppm.

2. Procédé selon la revendication 1, dans lequel la teneur en eau est comprise entre 50 et 1000 ppm.

3. Procédé selon la revendication 2, dans lequel la teneur en eau est comprise entre 250 et 600 ppm.

4. Procédé selon l'une des revendications 1 à 3, dans lequel l'agent nitrosant est le chlorure de nitrosyle.

5. Procédé selon l'une des revendications 1 à 2, dans lequel la concentration en cyclododécane dans le chloroforme est comprise entre 0,1 et 35 % en poids.

6. Procédé selon l'une des revendication 1 à 3, dans lequel la concentration en agent nitrosant est comprise entre 0,1 et 25 g/l de milieu réactionnel.

7. Procédé selon l'une des revendications 1 à 4, dans lequel le chlorure d'hydrogène est en excès par rapport à l'agent nitrosant.

## Patentansprüche

1. Verfahren zur Herstellung von Cyclododecanonoxim, das darin besteht, in Chloroform solubilisiertes Cyclododecan, ein Nitrosierungsreagens und Chlorwasserstoff unter solchen Bedingungen photochemisch umzusetzen, daß der Wassergehalt in dem Reaktionsmedium 1000 ppm nicht übersteigt.

2. Verfahren nach Anspruch 1, wobei der Wassergehalt im Bereich von 50 bis 1000 ppm liegt.

3. Verfahren nach Anspruch 2, wobei der Wassergehalt im Bereich von 250 bis 600 ppm liegt.

4. Verfahren nach einem der Ansprüche 1 bis 3, wobei das Nitrosierungsreagens das Nitrosylchlorid ist.

5. Verfahren nach einem der Ansprüche 1 bis 2, wobei die Konzentration an Cyclododecan in Chloroform im Bereich von 0,1 bis 35 Gew.-% liegt.

6. Verfahren nach einem der Ansprüche 1 bis 3, wobei die Konzentration des Nitrosierungsreagens im Bereich von 0,1 bis 25 g/l Reaktionsmedium liegt.

7. Verfahren nach einem der Ansprüche 1 bis 4, wobei der Chlorwasserstoff in bezug auf das Nitrosierungsreagens im Überschuß vorliegt.

## Claims

1. Process for the preparation of cyclododecanone oxime which consists in photochemically reacting cyclododecane, dissolved in chloroform, a nitrosating agent and hydrogen chloride under conditions such that the water content in the reaction medium does not exceed 1000 ppm.

2. Process according to Claim 1, in which the water content is between 50 and 1000 ppm.

3. Process according to Claim 2, in which the water content is between 250 and 600 ppm.

4. Process according to one of Claims 1 to 3, in which the nitrosating agent is nitrosyl chloride.

5. Process according to either of Claims 1 and 2, in which the concentration of cyclododecane in the chloroform is between 0.1 and 35% by weight.

6. Process according to one of Claims 1 to 3, in which the concentration of nitrosating agent is between 0.1 and 25 g/l of reaction medium.

7. Process according to one of Claims 1 to 4, in which the hydrogen chloride is in excess with respect to the nitrosating agent.
